Europäisches Patentamt

European Patent Office

Office européen des brevets

(11) Numéro de publication : **0 496 649 A1**

## (12) DEMANDE DE BREVET EUROPEEN

(21) Numéro de dépôt : **92400083.9**

(22) Date de dépôt : **13.01.92**

(51) Int. Cl.⁵ : **A61K 7/48,** A61K 35/78

(30) Priorité : **22.01.91 FR 9100697**

(43) Date de publication de la demande :
**29.07.92 Bulletin 92/31**

(84) Etats contractants désignés :
**AT BE CH DE DK ES FR GB GR IT LI LU MC NL
PT SE**

(71) Demandeur : **SYNTHELABO
22, Avenue Galilée
F-92350 Le Plessis Robinson (FR)**

(72) Inventeur : **Fabre, Bernard
4, rue du Pressois
F-37270 Saint-Martin-Le-Beau (FR)**
Inventeur : **Potier, Anne
39-41, rue Emile Zola, Appt. 10
F-37100 Tours (FR)**
Inventeur : **Geay, Béatrice
140, Avenue du Mans
F-37540 Saint-Cyr-sur-Loire (FR)**

(74) Mandataire : **Thouret-Lemaitre, Elisabeth et al
SYNTHELABO Service Brevets 22, avenue
Galilée
F-92352 LE PLESSIS ROBINSON CEDEX (FR)**

(54) **Extraits de feuilles d'Hamamélis, leur préparation et leurs applications.**

(57)    Extrait de feuilles d'Hamamélis, caractérisé en ce qu'il contient des tanins galliques, des acides phénoliques tels que l'acide gallique et l'acide caféique et des flavonoïdes, tels que le kaempférol, la quercétine et la quercitrine, la teneur moyenne en tanins galliques étant de 30 à 40 % et la teneur moyenne en acide gallique étant de 0,3 à 5 % par rapport au résidu sec de la solution extractive.

EP 0 496 649 A1

Jouve, 18, rue Saint-Denis, 75001 PARIS

La présente invention a pour objet des extraits de feuilles d'Hamamélis, leur préparation et leurs applications.

L'Hamamélis, Hamamélis virginiana L., est un arbre de 3 à 4 mètres de haut. Les feuilles sont entières et pétiolées. Le limbe est ovale, aigu au sommet, nettement asymétrique à la base.

La composition chimique des feuilles est la suivante :
– huiles essentielles (choline)
– acides phénoliques (acide gallique, acide caféique)
– phénols (catéchol)
– hétérosides flavoniques (kaempférol, quercétine et myricétine)
– tanins galliques (β-hamamélitanin et esters galliques du glucose).

Les extraits de feuilles d'Hamamélis obtenus selon le procédé de l'invention sont caractérisés par la présence de tanins galliques, par la présence de flavonoïdes tels que le kaempférol, la quercétine et la quercitrine, et par la présence d'acides phénoliques tels que acides gallique et caféique.

Les extraits de l'invention sont caractérisés par le fait qu'ils contiennent des tanins galliques, des acides phénoliques, tels que acides gallique et caféique, et des flavonoïdes, la teneur en tanins galliques étant de 30 à 40 % et la teneur en acide gallique étant de 0,3 à 5 % par rapport au résidu sec.

Selon l'invention, ou soumet les feuilles d'Hamamélis sèches ou fraîches, broyées ou pulvérisées, à une extraction par un solvant tel que l'eau, un alcanol en $C_1$-$C_4$, l'acétone ou le propylèneglycol, ou un mélange de ces solvants. Par exemple, on peut utiliser un mélange alcanol $C_1$-$C_4$/eau ou un mélange acétone/eau en proportions de 96/4 à 30/70 en volume. On peut également utiliser un mélange propylèneglycol/eau dans des proportions de 100/10 à 40/60 en volume.

La quantité de solvant d'extraction utilisée est égale à 4 à 20 fois le poids de feuilles d'Hamamélis. On effectue l'extraction éventuellement sous agitation à une température située entre 10°C et l'ébullition du solvant. La durée de l'extraction est de 2 à 80 heures.

On concentre éventuellement les solutions extractives. Les concentrats obtenus sont éventuellement séchés, soit en étuve sous vide, soit par nébulisation, soit par lyophilisation, soit par séchage à l'aide d'un sécheur-réacteur cylindrique.

Les extraits de feuilles d'Hamamélis obtenus selon le procédé de l'invention sont caractérisés par la présence de tanins galliques, par la présence de flavonoïdes tels que le kaempférol, la quercétine et la quercitrine, et par la présence d'acides phénoliques tels que acides gallique et caféique.

On caractérise les constituants des extraits de l'invention à l'aide des expériences suivantes :

On soumet une'solution éthanolique d'extraits de feuilles d'Hamamélis obtenue selon l'invention à la réaction à la cyanidine selon SHIBATA (Shibata. K., Nagai. I., Kishida. M., 1916, Journ. Biol. Chem. 28, p. 93). Cette réaction de référence, caractéristique des flavonoïdes à l'exception des chalcones et des isoflavones, s'effectue sur les extraits en solution à 5,0 % dans l'éthanol à 96 % v.v. Après agitation et filtration, on ajoute 3 ml d'acide chlorhydrique concentré à 5 ml du filtrat. L'addition de tournure de magnésidum provoque l'apparition d'une coloration rouge et d'un dégagement d'hydrogène caractéristiques des flavonoïdes. Cette coloration s'intensifie par ajout d'alcool amylique.

On met en présence une solution hydroalcoolique d'extraits de feuilles d'Hamamélis selon l'invention et une solution de cyanure de potassium (KCN). La solution se colore alors en orange, coloration caractéristique de l'acide gallique. La même solution donne ensuite un précipité caractéristique des tanins.

Une solution hydroalcoolique d'extraits de feuilles d'Hamamélis obtenue selon l'invention et contenant 10,0 % de chlorure ferrique (Fe2C16) précipite. Ce précipité, de couleur bleu-noir, est caractéristique des tanins galliques. Les flavanoïdes et l'acide gallique sont identifiés par chromatographie couche mince sur silice. La phase mobile est alors constituée d'un mélange de n-pentanol, d'hexane, d'acide acétique glacial et d'eau, dans une proportion 35-10 à 35-15. Après migration, la révélation par une solution de chlorure d'aluminium ou de chlorure ferrique à 2,0 % dans l'alcool permet d'identifier le kaempférol, la quercétine, la quercitrine et l'acide gallique. On dose les tanins par spectrophotométrie après réaction avec l'acide phosphotungstique, avant et après adsorption sur poudre de peau. Cette technique de dosage est développée dans la Pharmacopée française Xème édition pour le dosage des tanins dans les racines de ratanhia. Les teneurs en tanins dans les extraits obtenus selon l'invention varient de 30 à 40 % par rapport à la poudre pour un extrait sec, et par rapport au résidu sec pour un extrait liquide.

L'acide gallique est dosé par chromatographie liquide haute performance. La colonne utilisée est de type silice phase greffée C.18, la phase mobile est composée de proportions variables d'eau, de méthanol et d'acide acétique, le débit est de 1 millilitre par minute, la d'étection s'effectue à 254 nm.

Les teneurs en acide gallique varient de 0,3 à 5,0 % selon la nature des extraits obtenus selon l'invention.

Les exemples suivants illustrent l'invention.

Exemple 1.

On introduit dans un réacteur 1 kg de feuilles d'Hamamélis préalablement broyées et 10 litres d'eau. On porte le mélange à ébullition pendant 3 heures. On filtre, puis on concentre le filtrat sous vide à 60°C jusqu'à ce que l'on obtienne un concentrat de 1 kg. On sèche ce concentrat à l'étuve sous vide à 80°C, puis on le broie. On obtient une poudre marron foncé.

On met en évidence les flavonoïdes tels que le kaempférol, la quercétine et la quercitrine par chromatographie sur couche mince. On agit de même pour les acides phénoliques tels que l'acide gallique et l'acide caféique. On caractérise également ces substances par une réaction d'identification. Les tanins sont dosés par spectrophotométrie. Leur teneur moyenne est de 30 %. La teneur moyenne en acide gallique est de l'ordre de 5 %.

Exemple 2.

On introduit 500 grammes de feuilles d'Hamamélis préalablement broyées dans un réacteur agité avec 2,5 l d'éthanol à 30 %. On maintient l'agitation pendant 2 heures. Par filtration, on récupère la solution éthanolique, de couleur brune. Dans cette solution, on identifie par chromatographie sur couche mince les flavonoïdes et les acides phénoliques. On dose la teneur en tanins par dosage spectrophotométrique : elle est de 40 % en moyenne par rapport au résidu sec de la solution. La teneur moyenne en acide gallique est de 4,0 % par rapport au résidu sec de la solution.

On a testé l'activité des extraits de feuilles d'Hamamélis selon l'invention pour mettre en évidence une activité filtre U.V.B. et une activité anti-radicalaire.

L'activité filtre U.V.B. des extraits obtenus selon l'invention est démontrée à partir des observations suivantes : le spectre U.V. des extraits secs ou concentrés de feuilles d'Hamamélis solubilisés dans une solution aqueuse ou hydroalcoolique, ainsi que des extraits liquides, présente une zone d'absorption située entre 200 et 340 nm avec 2 maxima. Le premier situé entre 200 et 220 nm, le second entre 260 et 290 nm.

Ce dernier pic se situe dans la zone U.V.B. Les extraits obtenus selon l'invention absorbent donc dans la zone U.V.B. Ils peuvent donc être utilisés comme filtres U.V.B.

Les extraits obtenus selon l'invention présentent également des activités anti-radicalaires. Ces activités ont pu être démontrées in vitro par deux méthodes de dosage.

La première méthode d'évaluation a été réalisée sur un radical libre, le diphénylpicrylhydrazine hydrate qui absorbe dans le violet à 513 nm. (Lamaison J.L., Petitjean-Freytet C., Carnat P, Carnat A, 1988, Plantes Médicinales et Phytothérapie, 22, (4), p 231-234).

Un composé anti-radicalaire, piégeur de radicaux libres entraîne une diminution de la densité optique : l'activité anti-radicalaire d'une substance est exprimée en pourcentage de l'absorbance du diphénylpicrylhydrazine hydrate.

Dans ces conditions de test, un extrait de feuilles d'Hamamélis a une activité anti-radicalaire comparable à la vitamine E qui est la substance de référence la plus utilisée.

| Témoins | Concentration (mg/ml) | % Atténuation |
|---|---|---|
| ------ | | |
| Vitamine E | 0,01 | 9 |
| | 0,05 | 28 |
| | 0,1 | 61 |
| | 0,5 | 95 |
| | 1 | 96 |
| | | |
| Cynarine | 0,01 | 16 |
| | 0,05 | 60 |
| | 0,1 | 89 |
| | 0,5 | 94 |
| | 1 | 96 |
| | | |
| Extrait hydroalcoolique atomisé d'hamamélis | | |
| ----------------------- | 0,01 | 13 |
| | 0,05 | 43 |
| | 0,1 | 82 |
| | 0,5 | 95 |
| | 1 | 96 |

On a pu également évaluer l'activité anti-radicalaire sur l'anion superoxyde généré par la réaction du NADH (Nicotinamide Adénine Dinucléotique) sur le PMS (Phénazine Méthosulfate). (Roback J., Grylewski RJ, Biochemical Pharmacology, 1988, 37, (5), p 837-841). L'anion superoxyde provoque la réduction du NBT (Nitrobleu de Tétrazolium) en diformazan, de coloration bleue intense. On dose celui-ci par spectrophotométrie. On exprime l'activité anti-radicalaire d'une substance en pourcentage d'inhibition du développement de coloration due au diformazan. Par cette méthode de dosage, les extraits de feuilles d'Hamamélis présentent une activité anti-radicalaire marquée, deux fois supérieure en moyenne aux témoins testés, comme la quercétine ou l'acide rosmarinique.

| Témoins | Concentration (mg/ml) | % Inhibition |
|---|---|---|
| ------- | | |
| Quercétine | 0,01 | 1 |
| | 0,05 | 31 |
| | 0,1 | 69 |
| | 0,5 | 91 |
| | 1 | 98 |
| Acide rosmarinique | 0,01 | 2 |
| | 0,05 | 28 |
| | 0,1 | 59 |
| | 0,5 | 90 |
| | 1 | 99 |
| Extrait hydroalcoolique atomisé d'hamamélis | | |
| ----------------------- | 0,01 | 17 |
| | 0,05 | 62 |
| | 0,1 | 87 |
| | 1 | 95 |

Au vu de ces activités filtre U.V. et anti-radicalaire, on peut utiliser les extraits obtenus selon l'invention à des fins cosmétiques, en particulier pour la protection de la peau exposée à un ensoleillement prolongé, et pour le ralentissement du processus radicalaire de vieillissement.

Les tanins contenus dans les extraits de feuilles d'Hamamélis selon l'invention confèrent à ces extraits des propriétés antiprurigineuses, cicatrisantes, adoucissantes et émollientes pour lesquelles les feuilles d'Hamamélis sont utilisées traditionnellement en usage externe. Les extraits obtenus selon l'invention présentent en plus ces activités.

L'activité véinotonique des feuilles d'Hamamélis est bien connue. Cette activité est due aux substances phénoliques (tanins, flavonoïdes) contenues dans la plante. Ces substances sont présentes dans les extraits selon l'invention qui possèdent donc également une activité veinotonique.

Les extraits de feuilles d'Hamamélis selon l'invention peuvent être utilisés sous forme de pommade en association avec des excipients comme le monooléate de glycérol, la vaseline, la lanoline, la cire blanche, les huiles d'amande, de paraffine, l'eau réunis dans une même formule ou encore l'huile d'arachide, la lanoline anhydre, la glycérine, l'amidon de blé, la cire blanche et l'eau. Ils peuvent également rentrer dans la composition de crème dermique en association avec les excipients suivants : cire d'abeilles, paraffine liquide légère, eau, vaseline ou lanoline, cire blanche d'abeilles liquide légère, glycérol.

Des formules de produits solaires peuvent également contenir les extraits de l'invention s'ils sont incorporés dans des émulsions classiques H/E ou E/H ou plus simplement dans des supports huileux.

**Revendications**

1. Extrait de feuilles d'Hamamélis, caractérisé en ce qu'il contient des tanins galliques, des acides phénoliques tels que l'acide gallique et l'acide caféique et des flavonoïdes, tels que le kaempférol, la quercétine et la quercitrine, la teneur moyenne en tanins galliques étant de 30 à 40 % et la teneur moyenne en acide gallique étant de 0,3 à 5 % par rapport au résidu sec de la solution extractive.

2. Procédé d'obtention d'un extrait de feuilles d'Hamamélis selon la revendication 1, procédé caractérisé en ce que l'extrait est obtenu par extraction avec un solvant tel que l'eau, l'acétone, un alcanol en $C_1$-$C_4$, le propylèneglycol ou un mélange de ces solvants.

3. Procédé selon la revendication 2, caractérisé en ce que l'extraction est effectuée à l'aide d'un mélange alcanol en $C_1$-$C_4$/eau ou acétone/eau, en proportions de 96/4 à 30/70 en volume.

4. Procédé selon la revendication 2 caractérisé en ce que l'extraction est effectuée à l'aide d'un mélange propylèneglycol/eau, en proportions de 100/10 à 40/60 en volume.

5. Procédé selon l'une quelconque des revendications 2 à 4, caractérisé en ce que l'extraction est effectuée pendant une durée de 2 à 80 heures.

6. Procédé selon l'une quelconque des revendications 2 à 5, caractérisé en ce que l'extraction est statique ou agitée.

7. Procédé selon l'une quelconque des revendications 2 à 6, caractérisé en ce que la quantité de solvant utilisée égale 4 à 20 fois le poids de feuilles d'Hamamélis.

8. Composition cosmétique caractérisée en ce qu'elle contient un extrait selon la revendication 1, associé à un ou plusieurs excipients tels que le monooléate de glycérol, le glycérol, la vaseline, la lanoline, éventuellement anhydre, la cire blanche, la cire d'abeilles, les huiles d'amande et de paraffine, l'huile d'arachide, la glycérine, l'amidon de blé et l'eau.

Office européen
des brevets

# RAPPORT DE RECHERCHE EUROPEENNE

Numero de la demande

EP 92 40 0083

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | SEIFEN-ÖLE-FETTE-WACHSE, vol. 114, no. 1, janvier 1988, pages 15-16,18-20, Augsburg, DE; R. RILK: "Pflanzenextrakte - ein Naturgeheimnis?" * Page 18, colonne de droite, paragraphe 4 - page 20, colonne de droite, dernier paragraphe; page 16, parties 3,4 *<br>--- | 1-8 | A 61 K   7/48<br>A 61 K   35/78 |
| A | GB-A-2 095 553   (INTERAG RT) * En entier *<br>--- | 1-8 | |
| A | EP-A-0 133 151   (BERMAN) * En entier *<br>--- | 1,8 | |
| A | SEIFEN-ÖLE-FETTE-WACHSE, vol. 108, no. 15, septembre 1982, page A7, Augsburg, DE; "Cosmetochem." * En entier *<br>--- | 1-2 | |
| A | SEIFEN-ÖLE-FETTE-WACHSE, vol. 101, no. 14, 1975, page 393, Augsburg, DE; "Aerosolformulierungen mit Pflanzenextrakten" * Exemple: "Sonnenschutz-Spray" *<br>--- | 1-8 | DOMAINES TECHNIQUES RECHERCHES (Int. Cl.5)<br><br>A 61 K |
| A | STN SERVEUR DE BASES DE DONNEES, Karlsruhe, DE, FICHIER CHEMICAL ABSTRACTS, vol. 83, no. 16, résumé no. 136790d, Columbus, Ohio, US; P. BERNARD et al.: "Chemical composition of hazel leaves and its changes during water or water-alcohol extraction", & PLANT. MED. PHYTOTHER., 8(4), 255-62 * Résumé *<br>---                              -/- | 1-2 | |

Le présent rapport a été établi pour toutes les revendications

| Lieu de la recherche | Date d'achèvement de la recherche | Examinateur |
|---|---|---|
| LA HAYE | 27-03-1992 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

...............................................................

& : membre de la même famille, document correspondant

EPO FORM 1503 03.82 (P0402)

Office européen
des brevets

**RAPPORT DE RECHERCHE EUROPEENNE**

Numero de la demande

EP 92 40 0083

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | STN SERVEUR DE BASES DE DONNEES, Karlsruhe, DE, FICHIER BIOSIS, numéro d'acces AN=78:103887, Columbus, Ohio, US; P. BERNARD: "Hamamelis leaves", & PLANT MED. PHYTOTHER. 11 (SPEC. NO.). 1977 (RECD 1978), 184-188 * Résumé * ----- | 1-2 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| LA HAYE | 27-03-1992 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)